# EUROPEAN PATENT SPECIFICATION

(11) **EP 0 991 628 B1**
(45) Date of publication and mention of the grant of the patent: **12.01.2005**
(21) Application number: 98925022.0
(22) Date of filing: 28.05.1998
(51) Int. Cl.: C07D 241/44, C07D 405/10, C07D 215/20, C07D 215/38, A61K 31/47, A61K 31/495, C07D 405/12

(54) **QUINOLINE AND QUINOXALINE COMPOUNDS WHICH INHIBIT PLATELET-DERIVED GROWTH FACTOR AND/OR P56lck TYROSINE KINASES**
CHINOLIN- UND CHINOXALIN-VERBINDUNGEN DIE DEN VON BLUTPLÄTTCHEN ABSTAMMMENDEN WACHSTUMSFAKTOR UND/ODER PDGF- UND P56lck-TYROSIN-KINASE HEMMEN
QUINOLINE AND QUINOXALINE COMPOUNDS WHICH INHIBIT PLATELET-DERIVED GROWTH FACTOR AND/OR P56lck TYROSINE KINASES

(30) Priority: 28.05.1997 US 864455; 18.11.1997 US 972614
(43) Date of publication of application: 12.04.2000
(73) Proprietor: Aventis Pharmaceuticals Inc, Bridgewater, NJ 08807-0800 (US)
(72) Inventor: MYERS, Michael, Reading, PA 19606 (US); SPADA, Alfred, P., Lansdale, PA 19446 (US); PERSONS, Paul, E., Westborough, MA 01581 (US); MAGUIRE, Martin, P., Woburn, MA 01801 (US)
(74) Representative: Jones, Stephen Anthony
(86) International application number: PCT/US1998/010999
(87) International publication number: WO 1998/054156

(56) References cited:
- EP-A- 0 662 473
- US-A- 5 409 930
- US-A- 5 480 883
- US-A- 5 650 514
- MAGUIRE et al., "A New Series of PDGF Receptor Tyrosine Kinase Inhibitors: 3-Substituted Quinoline Derivatives", J. MED. CHEM., 08 July 1994, Vol. 37, No. 14, pages 2129-2137, XP002914345

## Description

### BACKGROUND OF THE INVENTION

### 1. Field of the Invention

This invention is directed to the inhibition of cell proliferation and/or cell matrix production and/or cell movement (chemotaxis) and/or T cell activation and proliferation using of quinoline/quinoxaline compounds which are useful protein tyrosine kinase inhibitors (TKIs).

Cellular signaling is mediated through a system of interactions which include cell-cell contact or cell-matrix contact or extracellular receptor-substrate contact. The extracellular signal is often communicated to other parts of the cell via a tyrosine kinase mediated phosphorylation event which affects substrate proteins downstream of the cell membrane bound signaling complex. A specific set of receptor-enzymes such as the insulin receptor, epidermal growth factor receptor (EGF-R) or platelet-derived growth factor receptor (PDGF-R) are examples of tyrosine kinase enzymes which are involved in cellular signaling. Autophosphorylation of the enzyme is required for efficient enzyme-mediated phosphorylation of substrate proteins containing tyrosine residues. These substrates are known to be responsible for a variety of cellular events including cellular proliferation, cellular matrix production. cellular migration and apoptosis to name a few.

It is understood that a large number of disease states are caused by either uncontrolled reproduction of cel ls or overproduction of matrix or poorly regulated programmed cell death (apoptosis). These disease states involve a variety of cell types and include disorders such as leukemia, cancer, glioblastoma, psoriasis, inflammatory diseases, bone diseases, fibrotic diseases, atherosclerosis and restenosis occurring subsequent to angioplasty of the coronary, femoral or kidney arteries or, fibroproliferative disease such as in arthritis, fibrosis of the lung, kidney and liver. In addition, deregulated cellular proliferative conditions follow from coronary bypass surgery. The inhibition of tyrosine kinase activity is believed to have utility in the control of uncontrolled reproduction of cells or overproduction of matrix or poorly regulated programmed cell death (apoptosis).

It is also known that certain tyrosine kinase inhibitors can interact with more than one type of tyrosine kinase enzyme. Several tyrosine kinase enzymes are critical for the normal function of the body. For instance, it would be undesirable to inhibit insulin action in most normal circumstances. Therefore, compounds which inhibit PDGF-R tyrosine kinase activity at concentrations less than the concentrations effective in inhibiting the insulin receptor kinase could provide valuable agents for the selective treatment of diseases characterized by cell proliferation and/or cell matrix production and/or cell movement (chemotaxis) such as restenosis.

This invention relates to the modulation and/or inhibition of cell signaling, cell proliferation, extracellular matrix production, chemotaxis. the control of abnormal cell growth and cell inflammatory response. More specifically. this invention relates to the use of substituted quinoxaline compounds which exhibit selective inhibition of differentiation, proliferation or mediator release by effectively inhibiting platelet-derived growth factor-receptor (PDGF-R) tyrosine kinase activity and/or Lck tyrosine kinase activity.

### 2. Reported Developments

A number of literature reports describe tyrosine kinase inhibitors which are selective for tyrosine kinase receptor enzymes such as EGF-R or PDGF-R or non-receptor cytosolic tyrosine kinase enzymes such as v-abl, p561ck or c-src. Recent reviews by Spada and Myers (*Exp. Opin. Ther. Patents* **1995**, *5*(8), 805) and Bridges *(Exp. Opin. Ther. Patents* **1995**, *5*(12), 1245) summarize the literature for tyrosine kinase inhibitors and EGF-R selective inhibitors respectively. Additionally Law and Lydon have summarized the anticancer potential of tyrosine kinase inhibitors *(Emerging Drugs: The Prospect For Improved Medicines* **1996**, 241-260).

Known inhibitors of PDGF-R tyrosine kinase activity includes quinoline-based inhibitors reported by Maguire et al. *(J. Med Chem.* **1994**, *37*,2129), and by Dolle et al. *(J. Med. Chem.* **1994**, *37*, 2627). A class of phenylamino-pyrimidine-based inhibitors was recently reported by Traxler et al. in EP 564409 and by Zimmerman, J.; and Traxler, P. et al. *(Biorg. & Med Chem. Lett.* 1996, *6*(11), 1221-1226) and by Buchdunger, E. et al. *(Proc. Nat. Acad. Sci.* **1995**. *92,* 2558). Despite the progress in the field there are no agents from these classes of compounds that have been approved for use in humans for treating proliferative disease.

The correlation between the multifactorial disease of restenosis with PDGF and PDGF-R is well-documented throughout the scientific literature. However, recent developments into the understanding of fibrotic diseases of the lung (Antoniades, H. N.: et al. *J. Clin. Invest.* **1990**, *86,* 1055). kidney and liver (Peterson, T. C. *Hepatology*,**1993**, *17*, 486) have also implicated PDGF and PDGF-R as playing a role. For instance glomerulonephritis is a major cause of renal failure and PDGF has been identified to be a potent mitogen for mesangial cells in vitro as demonstrated by Shultz et al. *(Am. J. Physiol.* **1988**, *255*, F674) and by Floege, et al. (*Clin. Exp. Immun.* **1991**, *86*, 334). It has been reported by Thornton, S. C.; et al. *(Clin. Exp. Immun.* **1991**, *86*, 79) that TNF-alpha and PDGF (obtained from human rheumatoid arthritis patients) are the major cytokines involved in proliferation of synovial cells. Furthermore, specific tumor cell types have been identified (see Silver, B. J., *BioFactors*, **1992,** *3*, 217) such as glioblastoma and Kaposi's sarcoma which overexpress either the PDGF protein or receptor thus leading to the uncontrolled growth of cancer cells via an autocrine or paracrine mechanism. Therefore, it is anticipated that a PDGF tyrosine kinase inhibitor would be useful in treating a variety of seemingly unrelated human disease conditions that can be characterized by the involvement of PDGF and or PDGF-R in their etiology.

The role of various non-receptor tyrosine kinases such as p56^{*lck*} (hereinafter "Lck") in inflammation-related conditions involving T cell activation and proliferation has been reviewed by Hanke, et al *(Inflamm. Res.* **1995**, *44*, 357) and by Bolen and Brugge (*Ann. Rev. Immunol*., **1997,** *15*, 371). These inflammatory conditions include allergy, autoimmune disease, rheumatoid arthritis and transplant rejection. Another recent review summarizes various classes of tyrosine kinase inhibitors including compounds having Lck inhibitory activity (Groundwater, et. al *Progress in Medicinal Chemistry,* **1996**, *33*, 233). Inhibitors of Lck tyrosine kinase activity include several natural products which are generally non-selective tyrosine kinase inhibitors such as staurosporine, genistein, certain flavones and erbstatin. Damnacanthol was recently reported to be a low nM inhibitor of Lck (Faltynek, et. al, *Biochemistry,* **1995**, *34*, 12464). Examples of synthetic Lck inhibitors include: a series of dihydroxy-isoquinoline inhibitors reported as having low micromolar to submicromolar activity (Burke, et al *J*. *Med. Chem.* **1993**, *36*, 425); and a quinoline derivative found to be much less active having an Lck IC₅₀ of 610 micromolar. Researchers have also disclosed a series of 4-substituted quinazolines that inhibit Lck in the low micromolar to submicromolar range (Myers et al, WO95/15758 and Myers, et. al *Bioorg. Med Chem. Lett.* **1997**, *7*, 417). Researchers at Pfizer (Hanke, et al *J. Biol. Chem.* **1996,** *271*, 695) have disclosed two specific pyrazolopyrimidine inhibitors known as PP1 and PP2 which have low nanomolar potency against Lck and Fyn. (another Src-family kinase). No Lck inhibitory has been reported regarding quinoline or quinoxaline based compounds. Therefore, it is anticipated that a quinoline or quinoxaline based inhibitor of Lck tyrosine kinase activity could be useful in treating a variety of seemingly unrelated human disease conditions that can be characterized by the involvement of Lck tyrosine kinase signaling in their etiology.

US 5,480,883 and US 5,409,930 disclose bis mono-and/or bicyclic aryl and heteroaryl compounds that are said to exhibit protein tyrosine kinase inhibition activity. EP-A-0662473 discloses 2-oxindole derivatives that are said to exhibit tyrosine kinase inhibition. A series of 3-substituted quinoline derivatives that are said to have the same activity are disclosed in J. Med. Chem. 1994, 37, 2129-2137.

### SUMMARY OF THE INVENTION

This invention is directed to a compound of formula I: 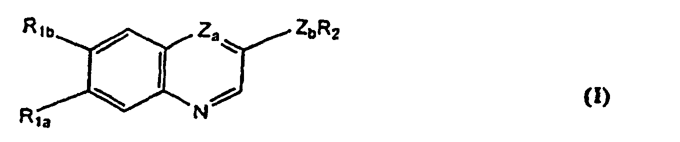 wherein
R₁ₐ is one to three carbon alkoxy;
R_{1b} is one to three carbon alkoxy;
R₂ is 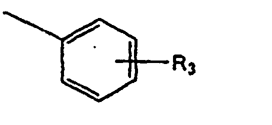 R₃ is hydrogen or meta- halo;
Zₐ is N or CH; and
Z_{b} is NH or O, or
an N-oxide thereof, hydrate thereof, solvate thereof, or salt thereof

Another aspect of the invention is directed to a pharmaceutical composition comprising a pharmaceutically effective amount of compound of fomula I and a pharmaceutically acceptable carrier.

### DETAILED DESCRIPTION OF THE INVENTION

As used above, and throughout the description of the invention, the following terms, unless otherwise indicated, shall be understood to have the following meanings:

### Definitions

"Patient" means a mammal including a human.

"Effective amount" means an amount of compound of the present invention effective in inhibiting PDGF-R tyrosine kinase activity and or Lck tyrosine kinase activity, and thus producing the desired therapeutic effect.

"Alkyl" means aliphatic hydrocarbon group which may be branched-or straight-chained having about 1 to about 10 carbon atoms. Preferred alkyl is "loweralkyl" having about 1 to about 3 carbon atoms; more preferred is methyl. Branched means that one or more lower alkyl groups such as methyl, ethyl or propyl are attached to a linear alkyl chain. The alkyl group is also optionally substituted by alkoxy. halo, carboxy, hydroxy or R₅R₆N- (wherein R₅ and R₆ are independently hydrogen or alkyl, or R₅ and R₆ taken together with the nitrogen atom to which R₅ and R₆ are attached form azaheterocyclyl); more preferably optionally substituted by fluoro. Examples of alkyl include methyl, fluoromethyl, difluoromethyl, trifluoromethyl, ethyl, n-propyl, isopropyl, butyl, sec-butyl, t-butyl, amyl and hexyl.

"Cycloalkyl" means a non-aromatic monocyclic ring system of about 3 to about 7 carbon atoms. Preferred mococyclic cycloalkyl rings include cyclopentyl, cyclohexyl and cycloheptyl; more preferred are cyclohexyl and cyclopentyl.

"Aryl" means aromatic carbocyclic radical containing about 6 to about 10 carbon atoms. Exemplary aryl include phenyl or naphthyl, or phenyl or naphthyl substituted with one or more aryl group substituents which may be the same or different where "aryl group substituent" includes hydrogen, hydroxy, halo, alkyl, alkoxy, carboxy, alkoxycarbonyl or Y¹Y²NCO-, wherein Y¹ and Y² are independently hydrogen or alkyl.

"Heteroaryl" means about a 5- to about a 10- membered aromatic monocyclic or multicyclic hydrocarbon ring system in which one or more of the carbon atoms in the ring system is/are element(s) other than carbon, for example nitrogen, oxygen or sulfur. The "heteroaryl" may also be substituted by one or more of the above-mentioned "aryl group substituents". Exemplary heteroaryl groups include substituted pyrazinyl, furanyl, thienyl, pyridyl, pyrimidinyl, isoxazolyl isothiazolyl, oxazolyl, thiazolyl, pyrazolyl, furazanyl, pyrrolyl, imidazo[2,1-b]thiazolyl, benzofurazanyl, indolyl, azaindolyl, benzimidazolyl, benzothienyl, quinolinyl, imidazolyl and isoquinolinyl.

"Heterocyclyl" means an about 4 to about 7 member monocyclic ring system wherein one or more of the atoms in the ring system is an element other than carbon chosen amongst nitrogen, oxygen or sulfur. The designation of the aza or oxa as a prefix before heterocyclyl define that at least a nitrogen, or oxygen atom is present respectively as a ring atom. Exemplary monocyclic heterocyclyl groups include piperidyl, pyrrolidinyl, piperazinyl, morpholinyl, thiomorpholinyl, thiazolidinyl, 1,3-dioxolanyl, 1,4-dioxanyl, tetrahydrofuranyl, tetrahydrothiophenyl, tetrahydrothiopyranyl, and the like. Exemplary heterocyclyl moieties include quinuclidyl, pentamethylenesulfide, tetrahydropyranyl, tetrahydrothiophenyl, pyrrolidinyl, tetrahydrofuranyl or 4-piperidinopiperidine.

"Heterocyclylcarbonyloxy" means a heterocyclyl-C(O)O- group wherein the heterocyclyl is as defined herein. An exemplary heterocyclylcarbonyloxy group is [1,4']-bipiperidin-1'-ylcarbonyloxy (4-piperidinopiperid-1-ylcarbonyloxy).

"Acyl" means an H-CO- or alkyl-CO- group in which the alkyl group is as previously described. Preferred acyls contain a lower alkyl. Exemplary acyl groups include formyl, acetyl, propanoyl, 2-methylpropanoyl, butanoyl and caproyl.

"Alkoxy" means an alkyl-O- group in which the alkyl group is as previously described. Preferred alkoxy is "lower alkoxy" having about 1 to about 3 carbon atoms; more preferred is methoxy. The alkoxy may be optionally substituted by one or more alkoxy, carboxy, alkoxycarbonyl, carboxyaryl or R₅R₆N- (wherein R₅ and R₆ are as defined above). Exemplary alkoxy groups include methoxy, ethoxy, *n*-propoxy, *i*-propoxy, *n*-butoxy, heptoxy, 2-(morpholin-4-yl)ethoxy and 2-(ethoxy)ethoxy.

"Cycloalkyloxy" means a cycloalkyl-O- group in which the cycloalkyl group is as previously described. Exemplary cycloalkyloxy groups include cyclopentyloxy or cyclohexyloxy.

"Heterocyclyloxy" means a heterocyclyl-O- group in which the heterocyclyl group is as previously described. Exemplary heterocyclyloxy groups include pentamethylenesulfideoxy. tetrahydropyranyloxy, tetrahydrothiophenyloxy, pyrrolidinyloxy or tetrahydrofuranyloxy.

"Aryloxy" means aryl-O- group in which the aryl group is as previously described.

"Heteroaryloxy" means heteroaryl-O- group in which the heteroaryl group is as previously described.

"Acyloxy" means an acyl-O- group in which the acyl group is as previously described.

"Carboxy" means a HO(O)C- (carboxylic acid) group.

"R₅R₆N-" means a substituted or unsubstituted amino group, wherein R₅ and R₆ are as previously described. Exemplary groups include amino (H₂N-), methylamino, ethylmethylamino, dimethylamino and diethylamino.

" R₅R₆NCO-" means a substituted or unsubstituted carbamoyl group, wherein R₅ and R₆ are as previously described. Exemplary groups are carbamoyl (H₂NCO-) and dimethylaminocarbamoyl (Me₂NCO-).

"AcylR₅ N-" means an acylamino group wherein R₅ and acyl are as defined herein.

"Halo" means fluoro, chloro, bromo, or iodo. Preferred are fluoro, chloro or bromo, and more preferred are fluoro or chloro.

"Prodrug" means a form of the compound of formula I suitable for administration to a patient without undue toxicity, irritation, allergic response. and the like, and effective for their intended use, including ketal, ester and zwitterionic forms. A prodrug is transformed *in vivo* to yield the parent compound of the above formula, for example by hydrolysis in blood. A thorough discussion is provided in T. Higuchi and V. Stella, Pro-drugs as Novel Delivery Systems, Vol. 14 of the A. C. S. Symposium Series, and in Edward B. Roche, ed., Bioreversible Carriers in Drug Design, American Pharmaceutical Association and Pergamon Press, 1987, both of which are incorporated herein by reference.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolable solvates. Representative solvates include ethanolates, methanolates, and the like. "Hydrate" is a solvate wherein the solvent molecule(s) is/are H₂O.

### Preferred Embodiments

A preferred compound aspect of the invention is a compound of formula I wherein R₁ₐ is methoxy or ethoxy.

Another preferred compound aspect of the invention is a compound of formula I wherein R_{1b} is methoxy or ethoxy.

Another preferred compound aspect of the invention is a compound of formula I wherein R₁ₐ and R_{1b} are lower alkoxy; more preferably the lower alkoxy is methoxy or ethoxy.

Another preferred compound aspect of the invention is a compound of formula I wherein R_{1c} is R_{1b} are methoxy or ethoxy.

Another preferred compound aspect of the invention is a compound of formula I wherein R₃ is hydrogen, or meta fluoro, chloro or bromo.

Another preferred compound aspect of the invention is a compound of formula wherem Zₐ is N.

Another preferred compound aspect of the invention is a compound of formula I wherein Zₐ is CH.

Another preferred compound aspect of the invention is a compound of formula I wherein Z_{b} is NH.

Another preferred compound aspect of the invention is a compound of formula I wherein Z_{b} is O.

Preferred compounds according to the invention are selected from the following species:
6,7-Diethoxy-2-phenoxyquinoxaline;
2-Phenylamino-6,7-diethoxyquinoxaline;
(6,7-Dimethoxyquinoxalin-2-yl)-(3-fluorophenyl)-amine;
2-(3-Fluorophenylamino)-6,7-diethoxyquinoxaline;
(3-Bromophenyl)-(6,7-dimethoxyquinoxalin-2-yl)-amine;
(6,7-Dimethoxyquinoxalin-2-yl)-phenyl-amine; and
(3-Chlorophenyl)-(6,7-dimethoxyquinoxalin-2-yl)-amine.

It is to be understood that this invention covers all appropriate combinations of the particular and preferred groupings referred to herein.

The compounds of this invention may be prepared by employing procedures known in the literature starting from known compounds or readily prepared intermediates. Exemplary general procedures follow.

In addition, compounds of formula I are prepared according to the following Schemes I-VI, wherein the variables are as described above, excepting those variables which one skilled in the art would appreciate would be incongruent with the method described.

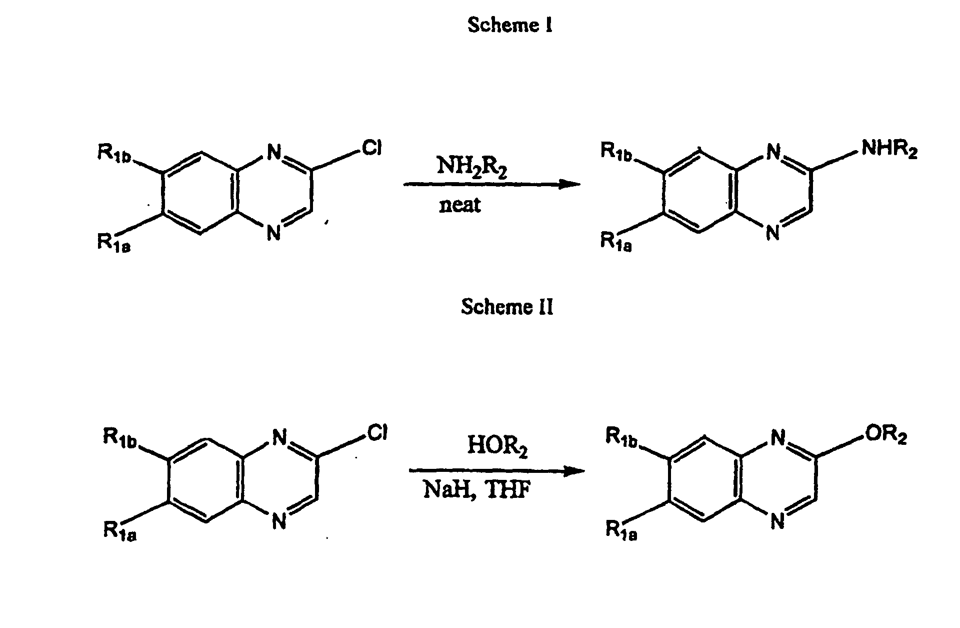

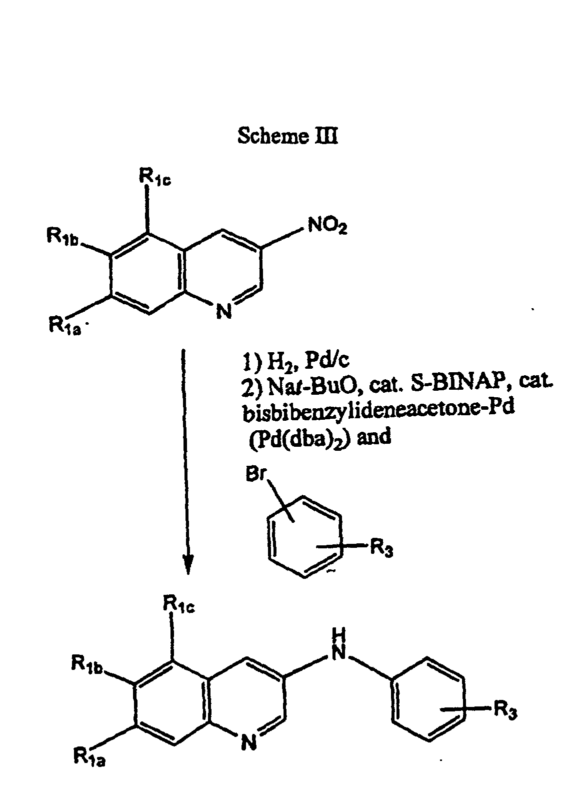

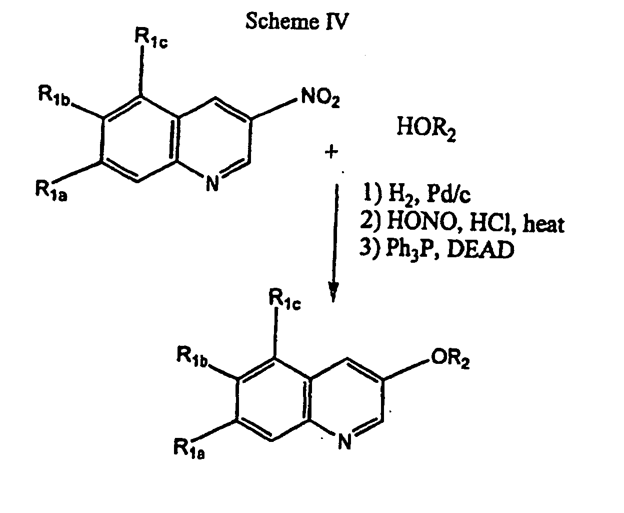

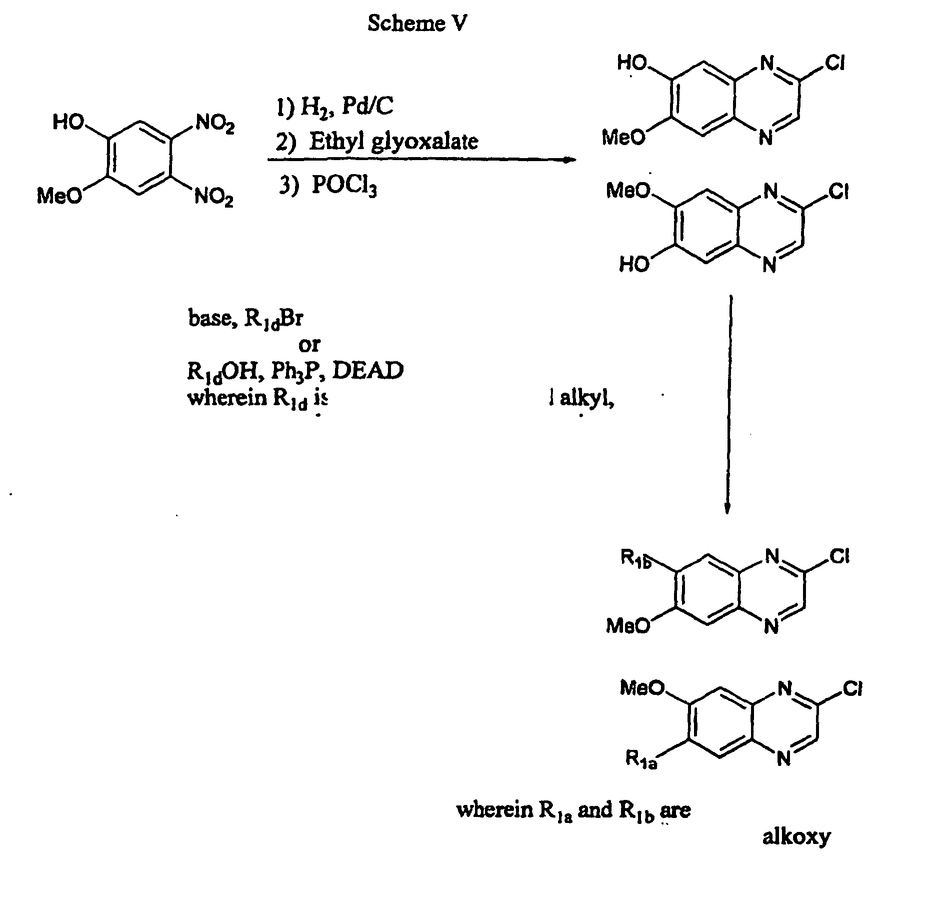

### I. General Procedures:

### 1. Coupling of 2-chloro substituted quinoxaline and amines or anilines

A mixture of 2-chloro-6,7-dimethoxyquinoxaline (1 eq.) and an amine (about 1 to about 5 eq.) is heated at about 160 to about 180°C from about three hours to overnight. The dark-brown residue is dissolved in methanol/ methylene chloride (0%-10%) and chromatographed on silica gel eluted with hexane/ethyl acetate or methanol/methylene chloride (0%-100%) to yield the desired product. The desired product may be purified further through recrystallization in methanol, methylene chloride or methanol/water.

### 2. Coupling of 2-chloro substituted quinoxaline and alcohols or phenols

A suspension of an alcohol or mercaptan (1 eq.) and sodium hydride (about 1 to about 3 eq.) in anhydrous DMF/THF (0%-50%) is refluxed for 1hour before addition of 2-chloro-6,7-dimethoxyquinoxaline (1 eq.). The resulting mixture is refluxed for about one to about four hours. The suspension is neutralized to about pH 5-8 and partitioned between methylene chloride and brine. The residue after concentration of methylene chloride is chromatographed on silica gel eluted with hexane/ethyl acetate or methanol/methylene chloride (0%-100%) to give the desired product.

### 3. Reductive animation reaction with amino-quinolines and aldehydes or ketones.

An appropriately substituted 3-amino quinoline (1 eq.) is stirred with 1 eq. of the appropriate aldehyde or ketone in methanol (or another suitable solvent mixture) until TLC indicates imine formation is complete. Excess NaCNBH₄ or NaBH₄, or another suitable reducing agent is added and the mixture is stirred until TLC shows consumption of the intermediate imine. The mixture is concentrated and the residue is chromatographed on silica gel with hexane/ethyl acetate (0-100 %) or chloroform/methanol (0-20%) to give the desired product.

### 4. coupling reaction of 3-amino substituted quinolines and bromophenyl compounds.

An appropriately substituted 3-amino quinoline (1 eq.) is stirred with ~1.4 eq. of a strong base such as sodium /-butoxide, 1 eq. of the appropriate bromophenyl compound, and catalytic amounts of 2,2'-bis(diphenylphosphino)-1-1'-binaphthyl (S-BINAP) and bis(dibenzylideneacetone)-Palladium (Pd(dba)₂) are mixed in an inert organic solvent such as toluene under an inert atmosphere such as argon and heated to about 80°C overnight. The mixture is cooled, diluted with a solvent such as ether, filtered. concentrated and chromatographed with 50% EtOAc/hexane, to give the desired product.

### 5. Ether formation from 3-hydroxy substituted quinolines via Mitsunobu conditions.

A THF solution of an appropriately substituted hydroxyquinoxaline (at about 0 to about 25 °C) is treated with 1 eq. each of the desired alcohol, triphenylphosphine and finally diethylazodicarboxylate (DEAD) or a suitable equivalent. The reaction progress is monitored via TLC and upon completion of the reaction (about 1 to about 24 hours) the mixture is concentrated and the residue is chromatographed on silica gel to yield the desired product.

### 6. Dealkylation of a lower alkoxy substituted quinoline or quinoxaline, and subsequent alkylation.

An appropriate lower alkoxy substituted quinoline or quinoxaline (1 eq.) in DMF is treated with excess sodium ethanthiolate (usually about 2 or more eq.) and the reaction mixture is stirred with heating from about 1 to about 24 hours. The mixture is partitioned between water and ethyl acetate. Extractive workup followed by chromatography, if necessary, provides the corresponding desired hydroxy substituted quinoline or quinoxaline product.

The hydroxy substituted quinoline or quinoxaline product can be alkylated using the conditions for the Mitsunobu reaction as detailed above. Alternatively, simple alkylation using methods wellknown in the art with a reactive alkyl- or benzyl- halide using NaH or another appropriate base in a suitable solvent provides the desired alkylated product.

### 7. Oxidation of a nitrogen in a quinoline or quinoxaline to the corresponding N-oxide.

An imine (=N-) moiety in a quinoline or quinoxaline compound of formula (I), may be converted to the corresponding compound wherein the imine moiety is oxidized to an N-oxide, preferably by reacting with a peracid, for example peracetic acid in acetic acid or m-chloroperoxybenzoic acid in an inert solvent such as dichloromethane, at a temperature from about room temperature to reflux, preferably at elevated temperature.

The compounds of the present invention are useful in the form of the free base or acid or in the form of a pharmaceutically acceptable salt thereof. All forms are within the scope of the invention.

Where the compound of the present invention is substituted with a basic moiety, acid addition salts are formed and are simply a more convenient form for use: and in practice, use of the salt form inherently amounts to use of the free base form. The acids which can be used to prepare the acid addition salts include preferably those which produce, when combined with the free base. pharmaceutically acceptable salts, that is, salts whose anions are non-toxic to the patient in pharmaceutical doses of the salts, so that the beneficial inhibitory effects on PDGF inherent in the free base are not vitiated by side effects ascribable to the anions. Although pharmaceutically acceptable salts of said basic compounds are preferred, all acid addition salts are useful as sources of the free base form even if the particular salt, per se, is desired only as an intermediate product as, for example, when the salt is formed only for purposes of purification, and identification, or when it is used as intermediate in preparing a pharmaceutically acceptable salt by ion exchange procedures. Pharmaceutically acceptable salts within the scope of the invention are those derived from the following acids: mineral acids such as hydrochloric acid, sulfuric acid, phosphoric acid and sulfamic acid; and organic acids such as acetic acid. citric acid, lactic acid, tartaric acid, malonic acid, methanesufonic acid, ethanesulfonic acid, benzenesulfonic acid, p-toluenesulfonic acid, cyclohexylsulfamic acid, quinic acid, and the like. The corresponding acid addition salts comprise the following: hydrohalides, e.g. hydrochloride and hydrobromide, sulfate, phosphate, nitrate, sulfamate, acetate, citrate, lactate, tartarate, malonate, oxalate, salicylate, propionate, succinate, fumarate, maleate, methylene-bis-(β-hydroxynaphthoates, gentisates, mesylates, isethionates and di-p-toluoyltartratesmethanesulfonate, ethanesulfonate, benzenesulfonate, p-toluenesulfonate, cyclohexylsulfamate and quinate, respectively.

According to a further feature of the invention, acid addition salts of the compounds of this invention are prepared by reaction of the free base with the appropriate acid, by the application or adaptation of known methods. For example, the acid addition salts of the compounds of this invention are prepared either by dissolving the free base in aqueous or aqueous-alcohol solution or other suitable solvents containing the appropriate acid and isolating the salt by evaporating the solution. or by reacting the free base and acid in an organic solvent, in which case the salt separates directly or can be obtained by concentration of the solution.

The compounds of this invention can be regenerated from the acid addition salts by the application or adaptation of known methods. For example, parent compounds of the invention can be regenerated from their acid addition salts by treatment with an alkali, e.g. aqueous sodium bicarbonate solution or aqueous ammonia solution.

Where the compound of the invention is substituted with an acidic moiety, base addition salts may be formed and are simply a more convenient form for use; and in practice, use of the salt form inherently amounts to use of the free acid form. The bases which can be used to prepare the base addition salts include preferably those which produce, when combined with the free acid, pharmaceutically acceptable salts, that is, salts whose cations are non-toxic to the animal organism in pharmaceutical doses of the salts, so that the beneficial inhibitory effects on PDGF inherent in the free acid are not vitiated by side effects ascribable to the cations. Pharmaceutically acceptable salts, including for example alkali and alkaline earth metal salts, within the scope of the invention are those derived from the following bases: sodium hydride, sodium hydroxide, potassium hydroxide, calcium hydroxide, aluminum hydroxide, lithium hydroxide, magnesium hydroxide, zinc hydroxide, ammonia, trimethylammonia, triethylammonia, ethylenediamine, *n*-methyl-glucamine, lysine, arginine, ornithine. choline, N,N'-dibenzylethylenediamine, chloroprocaine, diethanolamine, procaine, *n*-benzylphenethylamine, diethylamine, piperazine, tris(hydroxymethyl)-aminomethane. tetramethylammonium hydroxide, and the like.

Metal salts of compounds of the present invention may be obtained by contacting a hydride, hydroxide, carbonate or similar reactive compound of the chosen metal in an aqueous or organic solvent with the free acid form of the compound. The aqueous solvent employed may be water or it may be a mixture of water with an organic solvent, preferably an alcohol such as methanol or ethanol, a ketone such as acetone, an aliphatic ether such as tetrahydrofuran, or an ester such as ethyl acetate. Such reactions are normally conducted at ambient temperature but they may, if desired, be conducted with heating.

Amine salts of compounds of the present invention may be obtained by contacting an amine in an aqueous or organic solvent with the free acid form of the compound. Suitable aqueous solvents include water and mixtures of water with alcohols such as methanol or ethanol, ethers such as tetrahydrofuran, nitriles such as acetonitrile, or ketones such as acetone. Amino acid salts may be similarly prepared.

The compounds of this invention can be regenerated from the base addition salts by the application or adaptation of known methods. For example, parent compounds of the invention can be regenerated from their base addition salts by treatment with an acid, e.g., hydrochloric acid.

As well as being useful in themselves as active compounds, salts of compounds of the invention are useful for the purposes of purification of the compounds, for example by exploitation of the solubility differences between the salts and the parent compounds, side products and/or starting materials by techniques well known to those skilled in the art.

Compounds of the present invention may contain asymmetric centers. These asymmetric centers may independently be in either the R or S configuration. It will also be apparent to those skilled in the art that certain compounds of formula I may exhibit geometrical isomerism. Geometrical isomers include the *cis* and *trans* forms of compounds of the invention, i.e., compounds having alkenyl moieties or substituents on the ring systems. In addition, bicyclo ring systems include *endo* and *exo* isomers. The present invention comprises the individual geometrical isomers, stereoisomers, enantiomers and mixtures thereof.

Such isomers can be separated from their mixtures, by the application or adaptation of known methods, for example chromatographic techniques and recrystallization techniques, or they are separately prepared from the appropriate isomers of their intermediates, for example by the application or adaptation of methods described herein.

The starting materials and intermediates are prepared by the application or adaptation of known methods, for example methods as described in the Reference Examples or their obvious chemical equivalents, or by methods described according to the invention herein.

The present invention is further exemplified by the following illustrative examples which describe the preparation of the compounds according to the invention.

Further, the following examples are representative of the processes used to synthesize the compounds of this invention.

### REFERENCE EXAMPLE 1 2-Anilino-6-methoxy-quinoxaline hydrochloride

To 2-chloro-6-methoxy-quinoxaline (0.93 g, 4.8 mmol) under argon is added aniline (1.3 mL, 14.3 mmol). The reaction mixture is heated at 120°C for 2 hours, then at 150°C for 1.5 hours. The mixture is cooled and CH₂Cl₂ is added. The resulting suspension is stirred and the orange solid is filtered off, washed with CH₂Cl₂/Et₂O, then stirred vigorously in H₂O for 40 minutes, filtered, and washed with Et₂O to provide a bright-yellow solid.

The following compounds are prepared similarly beginning with the appropriate starting material.
2-(4-Fluorophenylamino)-6,7-diethoxyquinoxaline, m.p. 242°C. Anal. Calcd for C₁₈H₁₈FN₃O•0.50 H₂O: C, 64.27; H, 5.69; N, 12.49. Found: C, 64.21; H, 5.39; N, 12.24;
2-Phenylamino-6,7-diethoxyquinoxaline, m.p. 239°C;
(6,7-Dimethoxyqumoxalin-2-yl)-(3-fluorophenyl)-amine, m.p. 99-100°C, Anal. Calcd for C₁₆H₁₄FN₃O₂: C, 64.21; H, 4.71;F, 635; N, 14.04. Found: C, 64,35; H, 4.61; N, 13.84;
2-(3-Fluorophenylamino)-6,7-diethoxyquinoxaline, m.p. 193°C, Anal. Calcd for C₁₈H₁₈FN₃O₂•0.25 H₂O: C, 65.15; H, 5.62; N, 12.66. Found: C, 65.30; H, 5.30; N, 12.41;
(3-Bromophenyl)-(6,7-dimethoxyquinoxalin-2-yl)-amine, m.p. 197-198°C. Anal. Calcd for C₁₆H₁₄BrN₃O₂: C, 53.35; H, 3.92; Br, 22.18; N, 11.67. Found: C, 53.39; H, 3.82; N, 11.64;
(6,7-Dimethoxyquinoxalin-2-yl)-phenyl-amine, m.p. 88-90°C, Anal. Calcd for C₁₆H₁₅N₃O₂: C, 68.31; H, 5.37; N, 14.94. Found: C, 68.02; H, 5.52; N, 14.91; and
(3-Chlorophenyl)-(6,7-dimethoxyquinoxalin-2-yl)-amine, m.p. 187-188°C, Anal. Calcd for C₁₈H₁₄ClN₃O₂: C, 60.86; H, 4.47; Cl, 11.23; N, 13.31. Found: C, 60.85; H, 4.59; N, 13.26.

### REFERENCE EXAMPLE 2 2,7-Bis-cyclohexyloxy-6-methoxy-quinoxaline

To a DMF solution (5 mL) ofNaH (0.32 g, 8 mmol) under argon, cyclohexanol (0.7 mL, 6.7 mmol) is added dropwise. The mixture is stirred at room temperature for 25 minutes, then 2-chloro-6,7-dimethoxyquinoxaline is added portionwise. The reaction is stirred for 15 minutes at room temperature, at 90°C for 2 hours, and at 110°C for 1 hour. The mixture is cooled, quenched with H₂O, and partitioned between EtOAc/ H₂O. The organic layer is washed with H₂O and brine, dried (MgSO₄). and chromatographed (10% EtOAc/hexanes) to provide a waxy white solid (m.p. 75-78°C). Anal. Calcd. for C₂₁H₂₈N₂O₃: C, 70.76; H, 7.92; N, 7.86; Found: C, 70.81; H, 7.79; N, 7.70.

The following compound is prepared similarly beginning with the appropriate starting materials.
6,7-Diethoxy-2-phenoxyquinoxaline, m.p. 130-13 1°C. Anal. Calcd for C18H18N2O3: C, 69.66; H, 5.85; N, 9.03. Found: C, 69.53; H, 5.82; N, 8.91.

The compounds of formula I as described herein inhibit inhibition of cell proliferation and/or cell matrix production and/or cell movement (chemotaxis) via inhibition of PDGF-R tyrosine kinase activity. A large number of disease states are caused by either uncontrolled reproduction of cells or overproduction of matrix or poorly regulated programmed cell death (apoptosis). These disease states involve a variety of cell types and include disorders such as leukemia, cancer, glioblastoma. psoriasis, inflammatory diseases, bone diseases, fibrotic diseases, atherosclerosis and occurring subsequent to angioplasty of the coronary, femoral or kidney arteries or, fibroproliferative disease such as in arthritis, fibrosis ofthe lung, kidney and liver. In particular, PDGF and PDGF-R have been reported to be implicated in specific types of cancers and tumors such as brain cancer, ovarian cancer, colon cancer, prostate cancer lung cancer, Kaposi's sarcoma and malignant melanoma. In addition, deregulated cellular proliferative conditions follow from coronary bypass surgery. The inhibition of tyrosine kinase activity is believed to have utility in the control of uncontrolled reproduction of cells or overproduction of matrix or poorly regulated programmed cell death (apoptosis).

This invention relates to the modulation and/or inhibition of cell signaling, cell proliferation and/or cell matrix production and/or cell movement (chemotaxis), the control of abnormal cell growth and cell inflammatory response. More specifically, this invention relates to the use of substituted quinoline and quinoxaline compounds which exhibit selective inhibition of differentiation, proliferation. matrix production, chemotaxis or mediator release by effectively inhibiting platelet-derived growth factor-receptor (PDGF-R) tyrosine kinase activity.

Initiation of autophosphorylation, i.e., phosphorylation of the growth factor receptor itself, and of the phosphorylation of a host of intracellular substrates are some of the biochemical events which are involved in cell signaling, cell proliferation, matrix production, chemotaxis and mediator release.

By effectively inhibiting Lck tyrosine kinase activity, the compounds of this invention are also useful in the treatment of resistance to transplantation and autoimmune diseases such as rheumatoid arthritis, multiple sclerosis and systemic lupus erythematosus, in transplant rejection, in graft vs. host disease, in hyperproliferative disorders such as tumours and psoriasis, and in diseases in which cells receive pro-inflammatory signals such as asthma, inflammatory bowel disease and pancreatitis. In the treatment of resistance to transplantation, a compound of this invention may be used either prophylactically or in response to an adverse reaction by the human subject to a transplanted organ or tissue. When used prophylactically, a compound of this invention is administered to the patient or to the tissue or organ to be transplanted in advance of the transplantation operation. Prophylactic treatment may also include administration of the medication after the transplantation operation but before any signs of adverse reaction to transplantation are observed. When administered in response to an adverse reaction, a compound of this invention is administered directly to the patient in order to treat resistance to transplantation after outward signs of the resistance have been manifested.
According to a further feature of the invention there is provided the use of a compound of formula I in the preparation of a medicament for the treatment of a condition which may be ameliorated or prevented by the administration ofan inhibitor of PDGF-R tyrosine kinase activity and/or Lck tyrosine kinase activity, for example conditions as hereinbefore described.

Reference herein to treatment should be understood to include prophylactic therapy as well as treatment of established conditions.

The present invention also includes within its scope pharmaceutical compositions which comprise pharmaceutically acceptable amount of at least one of the compounds of formula I in association with a pharmaceutically acceptable carrier, for example, an adjuvant, diluent, coating and excipient.

In practice compounds or compositions for treating according to the present invention may administered in any variety of suitable forms, for example, by inhalation, topically, parenterally, rectally or orally; more preferably orally. More specific routes of administration include intravenous, intramuscular, subcutaneous, intraocular, intrasynovial, colonical, peritoneal, transepithelial including transdermal, ophthalmic, sublingual, buccal, dermal, ocular, nasal inhalation via insufflation, and aerosol.

The compounds of formula I may be presented in forms permitting administration by the most suitable route and the invention also relates to pharmaceutical compositions containing at least one compound according to the invention which are suitable for use as a medicament in a patient. These compositions may be prepared according to the customary methods, using one or more pharmaceutically acceptable adjuvants or excipients. The adjuvants comprise, inter alia, diluents, sterile aqueous media and the various non-toxic organic solvents. The compositions may be presented in the form of tablets, pills, granules, powders, aqueous solutions or suspensions, injectable solutions, elixirs or syrups, and may contain one or more agents chosen from the group comprising sweeteners such as sucrose, lactose. fructose, saccharin or Nutrasweet® , flavorings such as peppermint oil, oil of wintergreen. or cherry or orange flavorings, colorings, or stabilizers such as methyl- or propyl-paraben in order to obtain pharmaceutically acceptable preparations.

The choice of vehicle and the content of active substance in the vehicle are generally determined in accordance with the solubility and chemical properties of the product, the particular mode of administration and the provisions to be observed in pharmaceutical practice. For example, excipients such as lactose, sodium citrate, calcium carbonate, dicalcium phosphate and disintegrating agents such as starch, alginic acids and certain complex silica gels combined with lubricants such as magnesium stearate, sodium lauryl sulfate and talc may be used for preparing tablets, troches, pills, capsules and the like. To prepare a capsule, it is advantageous to use lactose and liquid carrier, such as high molecular weight polyethylene glycols. Various other materials may be present as coatings or to otherwise modify the physical form of the dosage unit. For instance, tablets, pills, or capsules may be coated with shellac, sugar or both. When aqueous suspensions are used they may contain emulsifying agents or agents which facilitate suspension. Diluents such as sucrose, ethanol, polyols such as polyethylene glycol, propylene glycol and glycerol, and chloroform or mixtures thereof may also be used. In addition, the active compound may be incorporated into sustained-release preparations and formulations.

For oral administration, the active compound may be administered, for example, with an inert diluent or with an assimilable edible carrier. or it may be enclosed in hard or soft shell gelatin capsules, or it may be compressed into tablets, or it may be incorporated directly with the food of the diet, or may be incorporated with excipient and used in the form of ingestible tablets, buccal tablets, troches, capsules, elixirs, suspensions, syrups, wafers, and the like.

For parenteral administration, emulsions, suspensions or solutions of the compounds according to the invention in vegetable oil, for example sesame oil, groundnut oil or olive oil, or aqueous-organic solutions such as water and propylene glycol, injectable organic esters such as ethyl oleate, as well as sterile aqueous solutions of the pharmaceutically acceptable salts, are used. The injectable forms must be fluid to the extent that it can be easily syringed, and proper fluidity can be maintained, for example, by the use of a coating such as lecithin, by the maintenance of the required particle size in the case of dispersion and by the use of surfactants. Prolonged absorption of the injectable compositions can be brought about by use of agents delaying absorption, for example, aluminum monostearate and gelatin. The solutions of the salts of the products according to the invention are especially useful for administration by intramuscular or subcutaneous injection. Solutions of the active compound as a free base or pharmacologically acceptable salt can be prepared in water suitably mixed with a surfactant such as hydroxypropyl-cellulose. Dispersion can also be prepared in glycerol, liquid polyethylene glycols, and mixtures thereof and in oils. The aqueous solutions, also comprising solutions of the salts in pure distilled water, may be used for intravenous administration with the proviso that their pH is suitably adjusted, that they are judiciously buffered and rendered isotonic with a sufficient quantity of glucose or sodium chloride and that they are sterilized by heating, irradiation, microfiltration, and/or by various antibacterial and antifungal agents, for example, parabens, chlorobutanol, phenol, sorbic acid, thimerosal, and the like.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique which yield a powder of the active ingredient plus any additional desired ingredient from previously sterile-filtered solution thereof.

Topical administration, gels (water or alcohol based), creams or ointments containing compounds of the invention may be used. Compounds of the invention may be also incorporated in a gel or matrix base for application in a patch, which would allow a controlled release of compound through *trans*dermal barrier.

For administration by inhalation, compounds of the invention may be dissolved or suspended in a suitable carrier for use in a nebulizer or a suspension or solution aerosol, or may be absorbed or adsorbed onto a suitable solid carrier for use in a dry powder inhaler.

Solid compositions for rectal administration include suppositories formulated in accordance with known methods and containing at least one compound of formula 1.

Compositions according to the invention may also be formulated in a manner which resists rapid clearance from the vascular (arterial or venous) wall by convection and/or diffusion, thereby increasing the residence time of the viral particles at the desired site of action. A periadventitial depot comprising a compound according to the invention may be used for sustained release. One such useful depot for administering a compound according to the invention may be a copolymer matrix, such as ethylene-vinyl acetate, or a polyvinyl alcohol gel surrounded by a Silastic shell. Alternatively, a compound according to the invention may be delivered locally from a silicone polymer implanted in the adventitia.

An alternative approach for minimizing washout of a compound according to the invention during percutaneous, transvascular delivery comprises the use of nondiffusible, drug-eluting microparticles. The microparticles may be comprised of a variety of synthetic polymers, such as polylactide for example, or natural substances, including proteins or polysaccharides. Such microparticles enable strategic manipulation of variables including total dose of drug and kinetics of its release. Microparticles can be injected efficiency into the arterial or venous wall through a porous balloon catheter or a balloon over stent, and are retained in the vascular wall and the periadventitial tissue for at least about two weeks. Formulations and methodologies for local, intravascular site-specific delivery of therapeutic agents are discussed in Reissen et al. *(J. Am. Coll. Cardiol.* 1994; 23: 1234-1244), the entire contents of which are hereby incorporated by reference.

A composition according to the invention may also comprise a hydrogel which is prepared from any biocompatible or non-cytotoxic (homo or hetero) polymer, such as a hydrophilic polyacrylic acid polymer that can act as a drug absorbing sponge. Such polymers have been described, for example, in application WO93/08845, the entire contents of which are hereby incorporated by reference. Certain of them, such as, in particular, those obtained from ethylene and/or propylene oxide are commercially available.

In the use of compounds according to the invention for treating pathologies which are linked to hyperproliferative disorders, the compounds according to the invention can be administered in different ways. For the treatment of restenosis, the compounds of the invention are administered directly to the blood vessel wall by means of an angioplasty balloon which is coated with a hydrophilic film (for example a hydrogel) which is saturated with the compound, or by means of any other catheter containing an infusion chamber for the compound, which can thus be applied in a precise manner to the site to be treated and allow the compound to be liberated locally and efficiently at the location of the cells to be treated. This method of administration advantageously makes it possible for the compound to contact quickly the cells in need of treatment.

The treatment method involving compounds of the invention preferably consists in introducing a compound according to the invention at the site to be treated. For example, a hydrogel containing composition can be deposited directly onto the surface of the tissue to be treated, for example during a surgical intervention. Advantageously, the hydrogel is introduced at the desired intravascular site by coating a catheter, for example a balloon catheter, and delivery to the vascular wall, preferably at the time of angioplasty. In a particularly advantageous manner, the saturated hydrogel is introduced at the site to be treated by means of a balloon catheter. The balloon may be chaperoned by a protective sheath as the catheter is advanced toward the target vessel, in order to minimize drug washoff after the catheter is introduced into the bloodstream.

The compound according to the invention may be administered by means of perfusion balloons. These perfusion balloons, which make it possible to maintain a blood flow and thus to decrease the risks of ischaemia of the myocardium, on inflation of the balloon, also enable the compound to be delivered locally at normal pressure for a relatively long time, more than twenty minutes, which may be necessary for its optimal action. Alternatively, a channelled balloon catheter ("channelled balloon angioplasty catheter", Mansfield Medical, Boston Scientific Corp., Watertown, MA) may be used. The latter consists of a conventional balloon covered with a layer of 24 perforated channels which are perfused via an independent lumen through an additional infusion orifice. Various types of balloon catheters, such as double balloon, porous balloon, microporous balloon, channel balloon, balloon over stent and hydrogel catheter, all of which may be used to practice the invention, are disclosed in Reissen et al. (1994).

The use of a perfusion balloon catheter is especially advantageous, as it has the advantages of both keeping the balloon inflated for a longer period of time by retaining the properties of facilitated sliding and of site-specificity of the hydrogel, are gained simultaneously.

Another aspect of the present invention relates to a pharmaceutical composition comprising a compound according to the invention and poloxamer, such as Poloxamer 407 is a non-toxic, biocompatible polyol, commercially available (BASF, Parsippany, NJ).

A poloxamer impregnated with a compound according to the invention may be deposited directly on the surface of the tissue to be treated, for example during a surgical intervention. Poloxamer possesses essentially the same advantages as hydrogel while having a lower viscosity.

The use of a channel balloon catheter with a poloxamer impregnated with a compound according to the invention is especially advantageous. In this case, the advantages of both keeping the balloon inflated for a longer period of time, while retaining the properties of facilitated sliding, and of site-specificity of the poloxamer, are gained simultaneously.

The percentage of active ingredient in the compositions of the invention may be varied, it being necessary that it should constitute a proportion such that a suitable dosage shall be obtained. Obviously, several unit dosage forms may be administered at about the same time. A dose employed may be determined by a physician or qualified medical professional, and depends upon the desired therapeutic effect, the route of administration and the duration of the treatment, and the condition of the patient. In the adult, the doses are generally from about 0.001 to about 50, preferably about 0.001 to about 5, mg/kg body weight per day by inhalation, from about 0.01 to about 100, preferably 0.1 to 70, more especially 0.5 to 10, mg/kg body weight per day by oral administration, and from about 0.001 to about 10, preferably 0.01 to 10, mg/kg body weight per day by intravenous administration. In each particular case, the doses are determined in accordance with the factors distinctive to the patient to be treated, such as age, weight, general state of health and other characteristics which can influence the efficacy of the compound according to the invention.

The compounds/compositions according to the invention may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of I to 4 doses per day, in accordance with the physiological requirements of each particular patient. Generally, the active product may be administered orally 1 to 4 times per day. Of course, for other patients. it will be necessary to prescribe not more than one or two doses per day.

The compounds of the present invention may also be formulated for use in conjunction with other therapeutic agents such as agents or in connection with the application of therapeutic techniques to address pharmacological conditions which may be ameliorated through the application of a compound of formula I, such as in the following:

The compounds of the present invention may be used in the treatment of restenosis post angioplasty using any device such as balloon, ablation or laser techniques. The compounds of the present invention may be used in the treatment of restenosis following stent placement in the vasculature either as 1) primary treatment for vascular blockage, or 2) in the instance where angioplasty using any device fails to give a patent artery. The compounds of the present invention may be used either orally, by parenteral administration or the compound could be applied topically through the intervention of a specific device or as a properly formulated coating on a stent device.

The compounds of the present invention may be used in the treatment of restenosis in combination with any anticoagulant, antiplatelet, antithrombotic or profibrinolytic agent. Often patients are concurrently treated prior, during and after interventional procedures with agents of these classes either in order to safely perform the interventional procedure or to prevent deleterious effects of thrombus formation. Some examples of classes of agents known to be anticoagulant, antiplatelet. antithrombotic or profibrinolytic agents include any formulation of heparin, low molecular weight heparins, pentasaccharides, fibrinogen receptor antagonists. thrombin inhibitors, Factor Xa inhibitors, or Factor VIIa inhibitors.

The compounds of the present invention may be used in combination with any antihypertensive agent or cholesterol or lipid regulating agent in the treatment of restenosis or atherosclerosis concurrently with the treatment of high blood pressure or atherosclerosis. Some examples of agents that are useful in the treatment of high blood pressure include compounds of the following classes; beta-blockers, ACE inhibitors, calcium channel antagonists and alpha-receptor antagonists. Some examples of agents that are useful in the treatment of elevated cholesterol levels or disregulated lipid levels include compounds known to be HMGCoA reductase inhibitors, compounds of the fibrate class,

The compounds of the present invention may be used in the treatment of various forms of cancer either alone or in combination with compounds known to be useful in the treatment of cancer.

It is understood that the present invention includes combinations of compounds of the present invention with one or more of the aforementioned therapeutic class agents

Compounds within the scope of the present invention exhibit marked pharmacological activities according to tests described in the literature which tests results are believed to correlate to pharmacological activity in humans and other mammals. The following pharmacological in vitro and in vivo test results are typical for characterizing compounds of the present invention.

### Preparation of Pharmaceutical Compositions and Pharmacological Test Section

Compounds within the scope of this invention exhibit significant activity as protein tyrosine kinase inhibitors and possess therapeutic value as cellular antiproliferative agents for the treatment of certain conditions including psoriasis, atherosclerosis and restenosis injuries. Compounds within the scope of the present invention exhibit the modulation and/or inhibition of cell signaling and/or cell proliferation and/or matrix production and/or chemotaxis and/or cell inflammatory response, and can be used in preventing or delaying the occurrence or reoccurrence of such conditions or otherwise treating the condition.

To determine the effectiveness of compounds of this invention, the pharmacological tests described below, which are accepted in the art and recognized to correlate with pharmacological activity in mammals, are utilized. Compounds within the scope of this invention have been subjected to these various tests, and the results obtained are believed to correlate to useful cellular differentiation mediator activity. The results of these tests are believed to provide sufficient information to persons skilled in the pharmacological and medicinal chemistry arts to determine the parameters for using the studied compounds in one or more of the therapies described herein.

### 1. PDGF-R Tyrosine Kinase Autophosphorylation ELISA assay

The titled assay is performed as described by Dolle et al. *(J. Med. Chem.* **1994**, *37*, 2627), which is incorporated herein by reference, with the exception of using the cell lysates derived from Human aortic smooth muscle cells (HAMSC) as described below.

### 2. Mitogenesis Assay General Procedure

### a. Cell Culture

Human aortic smooth muscle cells (passage 4-9) are plated in 96 well plates in a growth supporting medium at 6000 cells/well and allowed to grow 2-3 days.
At approximately 85% confluence. cells are growth arrested with serum free media (SFM).

### b. Mitogenesis Assay

After 24 hour serum deprivation, medium is removed and replaced with test compound/vehicle in SFM (200 µl/well). Compounds are solubilized in cell culture DMSO at a concentration of 10 mM and further dilutions are made in SFM.

After 30 min preincubation with compound, cells are stimulated with PDGF at 10 ng/mL. Determinations are performed in duplicate with stimulated and unstimulated wells at each compound concentration.

Four hours later, I µCi ³H thymidine/well is added.

Cultures are terminated 24 hours after addition of growth factor. Cells are lifted with trypsin and harvested onto a filter mat using an automated cell harvester (Wallac Mach 1196). The filter mat is counted in a scintillation counter (Wallac Betaplate) to determine DNA-incorporated label.

### 3. Chemotaxis Assay

Human aortic smooth muscle cells (HASMC) at earlier passages are obtained from ATCC. Cells are grown in Clonetics SmGM 2 SingleQuots (media and cells at passages 4-10 are used. When cells are 80% confluent, a fluorescent probe, calcein AM (5 mM, Molecular Probe), is added to the media and cells are incubated for 30 minutes. After washing with HEPES buffered saline, cells are lifted with trypsin and neutralized with MCDB 131 buffer (Gibco) with 0.1% BSA, 10 mM glutamine and 10% fetal bovine serum. After centrifugation, cells are washed one more time and resuspended in the same buffer without fetal bovine serum at 30000 cells/50 mL. Cells are incubated with different concentrations of a compound of formula I (final DMSO concentration = 1 %) for 30 min at 37°C. For chemotaxis studies, 96 well modified Boyden chambers (Neuroprobe, Inc.) and a polycarbonate membrane with 8 mm pore size (Poretics, CA) are used. The membrane is coated with collagen (Sigma C3657, 0.1 mg/mL). PDGF-ββ (3 ng/mL) in buffer with and without a compound of formula I are placed in the lower chamber. Cells (30,000), with and without inhibitor, are placed in the upper chamber. Cells are incubated for 4 hours. The tilter membrane is removed and cells on the upper membrane side are removed. After drying, fluoresce on the membrane is determined using Cytofluor II (Millipore) at excitation/emission wavelengths of 485/530 nm. In each experiment. an average cell migration is obtained from six replicates. Percent inhibition is determined from DMSO treated control values. From five points concentration-dependent inhibitions, IC₅₀ value is calculated. Results are presented as a mean±SEM from five such experiments.

### 4. EGF-Receptor Purification

EGF-receptor purification is based on the procedure of Yarden and Schlessinger. A431 cells are grown in 80 cm² bottles to confluency (2 x 10⁷ cells per bottle). The cells are washed twice with PBS and harvested with PBS containing 11.0 mmol EDTA (1 hour at 37°C, and centrifuged at 600g for 10 minutes. The cells are solubilized in 1 mL per 2 x 10⁷ cells of cold solubilization buffer (50 mmol Hepes buffer, pH 7.6, 1% Triton X- 100. 150 mmol NaCl, 5 mmol EGTA, I mmol PMSF, 50 mg/mL aprotinin. 25 mmol benzamidine, 5 mg/mL leupeptic, and 10 mg/mL soybean trypsin inhibitor) for 20 minutes at 4°C. After centrifugation at 100,000g for 30 minutes, the supernatant is loaded onto a WGA-agarose column (100 mL of packed resin per 2 x 10⁷ cells) and shaken for 2 hours at 4°C. The unabsorbed material is removed and the resin washed twice with HTN buffer (50 mmol Hepes, pH 7.6, 0.1% Triton X-100, 150 mmol NaCl), twice with HTN buffer containing 1 M NaCl, and twice with HTNG buffer (50 mmol Hepes, pH 7.6, 0. 1% Triton X-100. 150 mmol NaCl. and 10% glycerol). The EGF receptor is eluted batchwise with HTNG buffer containing 0.5 M N-acetyl-D-glucosamine (200 mL per 2 x 10⁷ cells.). The eluted material is stored in aliquots at -70°C and diluted before use with TMTNG buffer (50 mmol Tris-Mes buffer, pH 7.6, 0.1% Triton X- 100, 150 mmol NaCl, 10% glycerol).

### 5. Inhibition of EGF-R Autophosphorylation

A431 cells are grown to confluence on human fibronectin coated tissue culture dishes. After washing 2 times with ice-cold PBS. cells are lysed by the addition of 500 mL/ dish of lysis buffer (50 mmol Hepes. pH 7.5, 150 mmol NaCl, 1.5 mmol MgCl₂, 1 mmol EGTA, 10% glycerol. 1% triton X-100, 1 mmol PMSF, 1 mg/mL aprotinin, I mg/mL leupeptin) and incubating 5 minutes at 4°C. After EGF stimulation (500 mg/mL 10 minutes at 37°C) immunoprecipitation is performed with anti EGF-R (Ab 108) and the autophosphorylation reaction (50 mL aliquots, 3 mCi [g-³²P]ATP) sample is carried out in the presence of 2 or 10 mM of compound of the present invention, for 2 minutes at 4°C. The reaction is stopped by adding hot electrophoresis sample buffer. SDA-PAGE analysis (7.5% els) is followed by autoradiography and the reaction is quantitated by densitometry scanning of the x-ray films.

### a. Cell Culture

Cells termed HER 14 and K721A are prepared by *trans*fecting N1H3T3 cells (clone 2.2) (From C. Fryling, NCl, NIH), which lack *endo*genous EGF-receptors, with cDNA constructs of wild-type EGF-receptor or mutant EGF-receptor lacking tyrosine kinase activity (in which Lys 721 at the ATP-binding site is replace by an Ala residue, respectively). All cells are grown in DMEM with 10% calf serum (Hyclone, Logan, Utah).

### 6. Selectivity vs. PKA and PKC is determined using commercial kits:

### a. Pierce Colorimetric PKA Assay Kit. Spinzyme Format

Brief Protocol:
PKA enzyme (bovine heart) 1U/assay tube
Kemptide peptide (dye labeled) substrate
45 minutes @ 30°C
Absorbance at 570 nm

### b. Pierce Colorimetric PKC Assay kit, Spinzyme Format

Brief Protocol:
PKC enzyme (rat brain) 0.02SU/assay tube
Neurogranin peptide (dye labeled) substrate
30 minutes @ 30°C
Absorbance at 570 nm

### 7. p56^{lck}Tyrosine Kinase Inhibition Activity Measurements

p56^{*lck*} Tyrosine kinase inhibition activity is determined according to a procedure disclosed in United States Patent No. 5.714,493, incorporated herein by reference.

In the alternative, the tyrosine kinase inhibition activity is determined according to the following method. A substrate (tyrosine-containing substrate, Biot-(β Ala)₃-Lys-Val-Glu-Lys-fle-Gly-Glu-Gly-Thr-Tyr-Glu-Val-Val-Tyr-Lys-(NH₂) recognized by P56^{kk}, 1 µM) is first phosphorylated in presence or absence of a given concentration of the test compound, by a given amount of enzyme (enzyme is produced by expression of P56^{lck} gene in a yeast construct) purified from a cloned yeast (purification of the enzyme is done by following classical methods) in the presence of ATP (10µM) MgCl2( 2.5mM), MnCl2 (2.5mM), NaCl (25mM), DTT (0.4mM) in Hepes 50mM, pH 7.5, over 10 min at ambient temperature. The total reaction volume is 50µl, and the reactions are performed in a black 96-well fluoroplate. The reaction is stopped by addition of 150µl of stopping buffer (100mM Hepes pH7.5, KF 400mM, EDTA 133 mM, BSA 1g/l.) containing a selected anti tyrosine antibody labelled with the Europium cryptate (PY20-K) at 0.8µg/ml and allophycocyanine-labelled streptavidin (XL665) at 4µg/ml. The labelling of Streptavidin and anti-tyrosine antibodies were performed by Cis-Bio International (France). The mixture is counted using a Packard Discovery counter which is able to measure time-resolved homogeneous fluorescence transfer (excitation at 337 nm, readout at 620 nm and 665 nm). The ratio of the 665 nm signal / 620nm signal is a measure of the phosphorylated tyrosine concentration. The blank is obtained by replacing enzyme by buffer. The specific signal is the difference between the ratio obtained without inhibitor and the ratio with the blank. The percentage of specific signal is calculated. The IC₅₀ is calculated with 10 concentrations of inhibitor in duplicate using Xlfit soft. The reference compound is staurosporine (Sigma) and it exhibits an IC₅₀ of 30± 6 nM (n=20).

The results obtained by the above experimental methods. evidence that the compounds within the scope of the present invention possess useful PDGF receptor protein tyrosine kinase inhibition properties or p56^{lck} tyrosine kinase inhibition properties, and thus possess therapeutic value. The above pharmacological test results may be used to determine the dosage and mode of administration for the particular therapy sought.

## Claims

1. **A compound of Formula I** 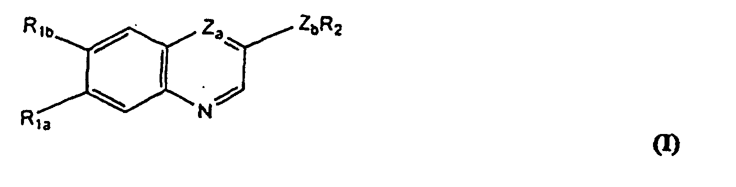 wherein
R₁ₐ is one to three carbon alkoxy;
R_{1b} is one to three carbon alkoxy;
R₂ is 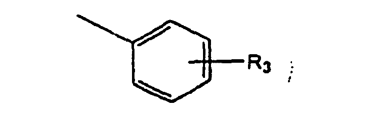 R₃ is hydrogen or meta- halo;
Zₐ is N or CH; and
Z_{b} is NH or O, or
an N-oxide thereof, hydrate thereof, solvate thereof, or salt thereof.

2. The compound of claim 1 wherein R₁ₐ is methoxy or ethoxy.

3. The compound of claim 1 wherein R_{1b} is methoxy or ethoxy.

4. The compound of claim 1 wherein R₁ₐ and R_{1b} are methoxy or ethoxy.

5. The compound of claim 1 wherein R₃ is hydrogen or meta-fluoro, meta-chloro or meta-bromo.

6. The compound of claim 1 wherein Zₐ is N.

7. The compound of claim 1, wherein Zₐ is CH.

8. The compound of claim 1 wherein Z_{b} is NH.

9. The compound of claim 1 wherein Z_{b} is O.

10. A compound of claim 1 which is selected from the following species:
6,7-Diethoxy-2-phenoxyquinoxaline;
2-Phenylamino-6,7-diethoxyquinoxaline;
(6,7-Dimethoxyquinoxalin-2-yl)-(3-fluorophenyl)-amine;
2-(3-Fluorophenylamino)-6,7-diethoxyquinoxaline;
(3-Bromophenyl)-(6,7-dimethoxyquinoxalin-2-yl)-amine;
(6,7-dimethoxyquinoxalin-2-yl)-phenyl-amine; and
(3-Chlorophenyl)-(6,7-dimethoxyquinoxalin-2-yl)-amine, or
an N-oxide thereof, hydrate thereof, solvate thereof, or pharmaceutically acceptable salt thereof.

11. The compound according to claim 1 which is (6,7-Dimethoxyquinoxalin-2-yl)-(3-fluorophenyl)-amine, or an N-oxide thereof, hydrate thereof, solvate thereof, or pharmaceutically acceptable salt thereof.

12. The compound according to claim 1 which is (3-Bromophenyl)-(6,7-dimethoxyquinoxalin-2-yl)-amine, or an N-oxide thereof, hydrate thereof, solvate thereof, or pharmaceutically acceptable salt thereof.

13. The compound according to claim 1 which is (3-Chlorophenyl)-(6,7-dimethoxyquinoxalin-2-yl)-amine, or an N-oxide thereof, hydrate thereof, solvate thereof, or pharmaceutically acceptable salt thereof.

14. A pharmaceutical composition comprising the compound of claim 1 and a pharmaceutically acceptable carrier.

15. A method for inhibiting PDGF tyrosine kinase activity comprising contacting a compound according to claim 1 with a composition ex-vivo containing a PDGF tyrosine kinase.

16. A method for inhibiting Lck tyrosine kinase activity comprising contacting a compound according to claim 1 with a composition ex-vivo containing a Lck tyrosine kinase.

17. Use of a compound according to claim 1 in the preparation of a medicament for inhibiting cell proliferation, differentiation, or mediator release in a patient suffering from a disorder **characterized by** such proliferation and/or differentiation and/or mediator release.

18. Use of a compound according to claim 1 in the preparation of a medicament used for treating a patient subject to a pathology linked to a hyperproliferative disorder.

19. Use according to claim 18, wherein said pathology is restenosis.

20. Use of a compound according to claim 1 in the preparation of a medicament for treating inflammation.

21. Use according to claim 19 wherein the compound according to claim 1 is incorporated into a coating on a stent device.

22. Use according to claim 18 wherein the pathology linked to a hyperproliferative disorder is a cancer susceptible to treatment by inhibition of PDGF tyrosine kinase.

23. Use according to claim 22 wherein the cancer is brain cancer, ovarian cancer, colon cancer, prostate cancer, lung cancer, Karposi's sarcoma or malignant melanoma.

24. Use according to claim 17 wherein the disorder is leukemia, cancer, glioblastoma, psoriasis, inflammatory diseases, bone diseases, fibrotic diseases, arthritis, fibrosis of the lung, fibrosis of the kidney, fibrosis of the liver, atherosclerosis; or restenosis occurring subsequent to angioplasty of the coronary, femoral or kidney arteries.

25. Use of a compound according to claim 1 in the preparation of a medicament for treating rheumatoid arthritis, multiple sclerosis, systemic lupus erythematosus, graft vs. host disease, asthma, inflammatory bowel disease or pancreatitis.

26. An angioplasty balloon coated with a hydrophilic film saturated with a compound according to claim 1.

27. A catheter comprising an infusion chamber containing a solution of a compound according to claim 1.

## Patentansprüche

1. Verbindung der Formel I 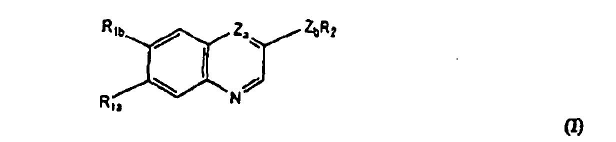 worin
R₁ₐ Alkoxy mit einem bis drei Kohlenstoffatomen darstellt; R_{1b} Alkoxy mit einem bis drei Kohlenstoffatomen darstellt;
R₂ 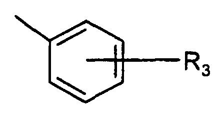 darstellt;
R₃ Wasserstoff oder meta-Halogen darstellt;
Zₐ N oder CH darstellt; und
Z_{b} NH oder O darstellt; oder
ein N-Oxid davon, Hydrat davon, Solvat davon oder Salz davon.

2. Verbindung nach Anspruch 1, worin R₁ₐ Methoxy oder Ethoxy darstellt.

3. Verbindung nach Anspruch 1, worin R_{1b} Methoxy oder Ethoxy darstellt.

4. Verbindung nach Anspruch 1, worin R₁ₐ und R_{1b} Methoxy oder Ethoxy darstellen.

5. Verbindung nach Anspruch 1, worin R₃ - Wasserstoff oder meta-Fluor, meta-Chlor oder meta-Brom darstellt.

6. Verbindung nach Anspruch 1, worin Zₐ N darstellt.

7. Verbindung nach Anspruch 1, worin Zₐ CH darstellt.

8. Verbindung nach Anspruch 1, worin Z_{b} NH darstellt.

9. Verbindung nach Anspruch 1, worin Z_{b} O darstellt.

10. Verbindung nach Anspruch 1, die ausgewählt ist aus den nachstehenden Spezies:
6,7-Diethoxy-2-phenoxychinoxalin;
2-Phenylamino-6,7-diethoxychinoxalin;
(6,7-Dimethoxychinoxalin-2-yl)-(3-fluorphenyl)-amin;
2-(3-Fluorphenylamino)-6,7-diethoxychinoxalin;
(3-Bromphenyl)-(6,7-dimethoxychinoxalin-2-yl)-amin;
(6,7-Dimethoxychinoxalin-2-yl)-phenyl-amin; und
(3-Chlorphenyl)-(6,7-dimethoxychinoxalin-2-yl)amin oder
ein N-Oxid davon, Hydrat davon, Solvat davon oder pharmazeutisch verträgliches Salz davon.

11. Verbindung nach Anspruch 1, die (6,7-Dimethoxychinoxalin-2-yl)-(3-fluorphenyl)-amin oder ein N-Oxid davon, Hydrat davon, Solvat davon oder pharmazeutisch verträgliches Salz davon darstellt.

12. Verbindung nach Anspruch 1, die (3-Bromphenyl)-(6,7-dimethoxychinoxalin-2-yl)-amin oder ein N-Oxid davon, Hydrat davon, Solvat davon oder pharmazeutisch verträgliches Salz davon darstellt.

13. Verbindung nach Anspruch 1, die (3-Chlorphenyl)-(6,7-dimethoxychinoxalin-2-yl)amin oder ein N-Oxid davon, Hydrat davon, Solvat davon oder pharmazeutisch verträgliches Salz davon darstellt.

14. Pharmazeutische Zusammensetzung, umfassend die Verbindung nach Anspruch 1 und einen pharmazeutisch verträglichen Träger.

15. Verfahren zum Inhibieren der PDGF-Tyrosinkinase-Aktivität, umfassend In-Kontakt-Bringen einer Verbindung nach Anspruch 1 ex-vivo mit einer Zusammensetzung, die eine PDGF-Tyrosinkinase enthält.

16. Verfahren zum Inhibieren der Lck-Tyrosinkinase-Aktivität, umfassend In-Kontakt-Bringen einer Verbindung nach Anspruch 1 ex-vivo mit einer Zusammensetzung, die eine Lck-Tyrosinkinase enthält.

17. Verwendung einer Verbindung nach Anspruch 1 bei der Herstellung eines Arzneimittels zum Inhibieren von Zellproliferation, Differentierung oder Mediatorfreisetzung bei einem Patienten, der unter einer Störung, die durch solche Proliferation und/oder Differentierung und/oder Mediatorfreisetzung gekennzeichnet ist, leidet.

18. Verwendung einer Verbindung nach Anspruch 1 bei der Herstellung eines Arzneimittels, das zum Behandeln eines Patienten verwendet wird, der für eine mit einer hyperproliferativen Störung verbundenen Pathologie veranlagt ist.

19. Verwendung nach Anspruch 18, wobei die Pathologie Restenose ist.

20. Verwendung einer Verbindung nach Anspruch 1 bei der Herstellung eines Arzneimittels zum Behandeln von Entzündung.

21. Verwendung nach Anspruch 19, wobei die Verbindung nach Anspruch 1 in eine Beschichtung auf einer Stent-Vorrichtung eingearbeitet wird.

22. Verwendung nach Anspruch 18, wobei die mit einer hyperproliferativen Störung verbundene Pathologie ein Krebs ist, der für die Behandlung durch Inhibierung von PDGF-Tyrosin-kinase anfällig ist.

23. Verwendung nach Anspruch 22, wobei der Krebs Hirnkrebs, Eierstockkrebs, Darmkrebs, Prostatakrebs, Lungenkrebs, Karposi-Sarcom oder maligne Melanome ist.

24. Verwendung nach Anspruch 17, wobei die Störung Leukämie, Krebs, Glioblastom, Psoriasis, entzündliche Erkrankungen, Knochenerkrankungen, fibrotische Erkrankungen, Arthritis, Fibrose der Lunge, Fibrose der Niere, Fibrose der Leber, Atherosklerose; oder Restenose, die anschließend an Angioplastie der koronaren, femoralen oder Nierenarterien auftritt, ist.

25. Verwendung einer Verbindung nach Anspruch 1 bei der Herstellung eines Arzneimittels zum Behandeln von rheumatischer Arthritis, multipler Sklerose, systemischem Lupus erythematosus, Transplantat-Empfänger-Abstoßungsreaktion, Asthma, entzündlicher Darmerkrankung oder Pankreatitis.

26. Angioplastischer Ballon, beschichtet mit einem hydrophilen Film, der mit einer Verbindung nach Anspruch 1 gesättigt ist.

27. Katheter, umfassend eine Infusionskammer, die eine Lösung einer Verbindung nach Anspruch 1 enthält.

## Revendications

1. Composé singulier de formule I 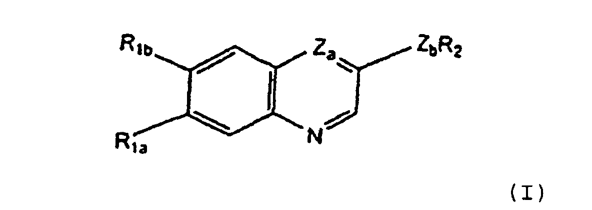 dans laquelle
R₁ₐ est un alcoxy à un à trois atomes de carbone ;
R_{1b} est un alcoxy à un à trois atomes de carbone ;
R₂ est 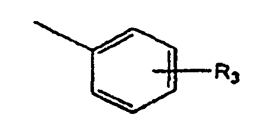 R₃ est un hydrogène ou un méta-halogéno ;
Zₐ est N ou CH ; et
Z_{b} est NH ou O, ou
un N-oxyde de celui-ci, un hydrate de celui-ci, un solvate de celui-ci, ou un sel de celui-ci.

2. Composé selon la revendication 1, dans lequel R₁ₐ est un méthoxy ou un éthoxy.

3. Composé selon la revendication 1, dans lequel R_{1b} est un méthoxy ou un éthoxy.

4. Composé selon la revendication 1, dans lequel R₁ₐ et R_{1b} sont un méthoxy ou un éthoxy.

5. Composé selon la revendication 1, dans lequel R₃ est un hydrogène ou un méta-fluoro, un méta-chloro ou un méta-bromo.

6. Composé selon la revendication 1, dans lequel Zₐ est N.

7. Composé selon la revendication 1, dans lequel Zₐ est CH.

8. Composé selon la revendication 1, dans lequel Z_{b} est NH.

9. Composé selon la revendication 1, dans lequel Z_{b} est O.

10. Composé selon la revendication 1, qui est choisi parmi les espèces suivantes :
la 6,7-diéthoxy-2-phénoxyquinoxaline ;
la 2-phénylamino-6,7-diéthoxyquinoxaline ;
la (6,7-diméthoxyquinoxalin-2-yl)(3-fluorophényl)amine ;
la 2-(3-fluorophénylamino)-6,7-diéthoxyquinoxaline ;
la (3-bromophényl)-(6,7-diméthoxyquinoxalin-2-yl)amine ;
la (6,7-diméthoxyquinoxalin-2-yl)phényl-amine ; et
la (3-chlorophényl)-(6,7-diméthoxyquinoxalin-2-yl)amine, ou
un N-oxyde de celui-ci, un hydrate de celui-ci, un solvate de celui-ci, ou un sel pharmaceutiquement acceptable de celui-ci.

11. Composé selon la revendication 1, qui est la (6,7-diméthoxyquinoxalin-2-yl)-(3-fluorophényl)amine, ou un N-oxyde de celle-ci, un hydrate de celle-ci, un solvate de celle-ci, ou un sel pharmaceutiquement acceptable de celle-ci.

12. Composé selon la revendication 1, qui est la (3-bromophényl)-(6,7-diméthoxyquinoxalin-2-yl)amine, ou un N-oxyde de celle-ci, un hydrate de celle-ci, un solvate de celle-ci, ou un sel pharmaceutiquement acceptable de celle-ci.

13. Composé selon la revendication 1, qui est la (3-chlorophényl)-6,7-diméthoxyquinoxalin-2-yl)amine,
ou un N-oxyde de celui-ci, un hydrate de celui-ci, un solvate de celui-ci, ou un sel pharmaceutiquement acceptable de celui-ci.

14. Composition pharmaceutique comprenant le composé selon la revendication 1 et un support pharmaceutiquement acceptable.

15. Méthode d'inhibition de l'activité tyrosine kinase de PDGF, comprenant la mise en contact d'un composé selon la revendication 1 avec une composition ex-vivo contenant une tyrosine kinase de PDGF.

16. Méthode d'inhibition de l'activité tyrosine kinase de Lck, comprenant la mise en contact d'un composé selon la revendication 1 avec une composition ex-vivo contenant une tyrosine kinase de Lck.

17. Utilisation d'un composé selon la revendication 1 pour la préparation d'un médicament destiné à l'inhibition de la prolifération, de la différenciation ou de la libération de médiateur cellulaire chez un patient souffrant d'un trouble **caractérisé par** une telle prolifération et/ou différenciation et/ou libération de médiateur.

18. Utilisation d'un composé selon la revendication 1 pour la préparation d'un médicament utilisé pour le traitement d'un patient sujet à une pathologie liée à un trouble hyperprolifératif.

19. Utilisation selon la revendication 18, dans laquelle ladite pathologie est la resténose.

20. Utilisation d'un composé selon la revendication 1 pour la préparation d'un médicament destiné au traitement d'une inflammation.

21. Utilisation selon la revendication 19, dans laquelle le composé selon la revendication 1 est incorporé dans un enrobage sur un dispositif à-stent.

22. Utilisation selon la revendication 18, dans laquelle la pathologie liée à un trouble hyperprolifératif est un cancer susceptible d'être traité par inhibition de la tyrosine kinase de PDGF.

23. Utilisation selon la revendication 22, dans laquelle le cancer est le cancer du cerveau, le cancer de l'ovaire, le cancer du colon, le cancer de la prostate, le cancer du poumon, le sarcome de Karposi ou un mélanome malin.

24. Utilisation selon la revendication 17, dans laquelle le trouble est la leucémie, le cancer, le glioblastome, le psoriasis, des maladies inflammatoires, des maladies osseuses, des maladies fibrotiques, l'arthrite, la fibrose du poumon, la fibrose du rein, la fibrose du foie, l'athérosclérose ; ou la resténose se produisant par suite d'une angioplastie des artères coronaire, fémorale ou rénale.

25. Utilisation selon la revendication 1, pour la préparation d'un médicament destiné au traitement de la polyarthrite rhumatoïde, de la sclérose en plaques, du lupus érythémateux systémique, de la maladie de la greffe contre l'hôte, de l'asthme, de la maladie intestinale inflammatoire ou de la pancréatite.

26. Ballon d'angioplastie enrobé d'un film hydrophile saturé en un composé selon la revendication 1.

27. Cathéter comprenant une enceinte de perfusion contenant une solution d'un composé selon la revendication 1.
